# EUROPEAN PATENT APPLICATION

(11) **EP 2 436 673 A1**
(43) Date of publication of application: **04.04.2012**
(21) Application number: 11007990.2
(22) Date of filing: 30.09.2011
(51) Int. Cl.: C07D 231/12, C07D 403/12, A61K 9/127, A61K 31/415, A61K 31/4192, A61P 25/00

(54) **Curcumin derivatives with improved physicochemical properties and nanoliposomes surface-decorated with the derivatives with very high affinity for amyloid-beta1-42 peptide**

(30) Priority: 30.09.2010 GR 2010100563
(71) Applicant: Niaraki, Anna, 26504 Arachovitika Patron (GR); University Of Patras, 26500 Patra (GR); Università degli Studi di Milano - Bicocca, 20126 Milano (IT); Antimisiari Sofia, 26442 Patra (GR); Mourtas, Spiridon, 26441 Patra (GR)
(72) Inventor: Antimisiari, Sophia, 26442 Patra (GR); Mourtas, Spiridon, 26441 Patra (GR); Niaraki, Anna, 26504 Arachovitika Patron (GR); Zona, Cristiano, 20126 Milano (IT); Masserini, Massimo, Milano (IT); Nicotra, Francesco, 20126 Milano (IT); La Ferla, Barbara, 20126 Milano (IT)
(74) Representative: Malamis, Alkisti-Irene

(57) **Abstract**

Amyloid β (Aβ) aggregates are considered as possible targets for therapy and/or diagnosis of Alzheimer disease (AD). It has been previously shown that curcumin targets Aβ plaques and interferes with their formation, suggesting a potential role for prevention or treatment of AD. In the present invention, curcumin-derivatives with improved physicochemical properties were synthesized and a "click chemistry" as well as a conventional liposome preparation method, were used to generate nanoliposomes decorated with the curcumin derivatives. These derivatives were designed to maintain the planar structure required for interaction with Aβ, as directly confirmed by Surface Plasmon Resonance experiments. Surface Plasmon Resonance experiments, measuring the binding of flowing liposomes to immobilized Aβ1-42, indicated that the liposomes exposing curcumin derivatives have extremely high affinity for Aβ1-42 fibrils (1-5 nM), likely because of the occurrence of multivalent interactions. The present invention describes the synthesis of the curcumin derivatives and the preparation and characterization of new nanoliposomes with a very high affinity for Aβ1-42 fibrils, to be exploited as vectors for the targeted delivery of new diagnostic and therapeutic molecules for AD.

## Description

**Field of the invention** - The present invention applies to the field of synthesis of novel curcumin derivatives and functionalization of liposomes with the aim to provide a vector for the targeted delivery of new diagnostic and therapeutic molecules for Alzheimer's disease (AD).

### PRIOR ART

Alzheimer's disease (hereinafter referred to as AD) is the most common form of dementia among neurodegenerative diseases in the elderly population, and the fourth common cause of death in Western countries after heart disease, cancer and stroke. More than 5 million Americans and 3 million Europeans are believed to have AD, while these numbers are estimated to increase to 15 million and more than 6 million in the next 40 years, respectively, due to increase of the elderly population.

The mechanisms underlying AD are not yet completely clear, and there is still no cure. However, in recent years, several approaches aimed at inhibiting disease progression have advanced to clinical trials. Among these, strategies targeting the production and clearance of the amyloid-beta (hereinafter referred to as Aβ) peptide are the most advanced. Indeed, genetic, pathological and biochemical clues suggest that the progressive production and subsequent accumulation of Aβ, plays a central role in AD pathogenesis. The predominant and initial peptide deposited in the brain parenchyma is Aβ1-42, a highly fibrillogenic peptide which is believed to follow metal induced aggregation to form plaques. Oligomers appearing before plaque deposition in an early stage of AD pathology have been indicated as the most toxic Aβ species. Targeting Aβ1-42 in all its aggregation forms has been suggested for therapeutic and/or diagnostic purposes. Moreover, it has been recently demonstrated that brain and blood Aβ are in equilibrium through the blood brain barrier (BBB), and sequestration of Aβ in the blood may shift this equilibrium, drawing out the excess from the brain ("sink" effect). According to this finding, blood circulating Aβ peptides could also be potential targets for the therapy of AD.

Curcumin is a naturally occurring phytochemical. It has been shown to be a potent anti-oxidant and anti-inflammatory compound with a favorable toxicity profile; and a free (nitric oxide-based) radical scavenger, which protects the brain from lipid peroxidation. Curcumin inhibits amyloid Aβ1-42 oligomer formation and cell toxicity at nanomolar concentrations *in vitro,* and binds to plaques reducing amyloid levels *in vivo.* Thus, it has been hypothesized that its extensive use might account for the significantly lower prevalence of AD in the Asian-Indian population and that curcumin treatment might be beneficial for AD patients.

Curcumin is stable at acidic and high pH (> 11.7) but unstable at neutral and basic pH and it is degraded to (5E)-6-(4'-hydroxy-3'-mothoxyphenyl)-2,4-dioxo-5-hexenal as major product and vanillin, ferulic acid and feruloylmethane. In fact, most curcumin (> 90%) is rapidly degraded within 30 min of placement in phosphate buffer systems of pH 7.2. In comparison, curcumin is more stable in cell culture medium containing 10 % fetal calf serum and in human blood, < 20% of curcumin being degraded within 1 h and approximately 50% by 8 h . In water environment Curcumin exists in enolic and β-diketonic forms, due to its cheto-enol tautomerism. The fact that curcumin in solution exists primarily in its enolic form has an important bearing on the radical-scavenging ability of curcumin and on its Abeta-binding activities .

In 2001, Lim et al, suggested that curcumin was able to block Alzheimer's disease pathogenesis at multiple sites of the inflammation cascade, but the direct effects of curcumin on the formation and destabilization of Abeta remain unclear. In 2007, Reinke et al. studied the structure-activity relationships of amyloid beta-aggregation inhibitors based on curcumin. They studied the effect of three prominent features on inhibition of amyloid aggregation: the presence of two aromatic end groups, the substitution pattern of these aromatics, and the length and flexibility of the linker region. They noticed that the two aromatic rings need to be coplanar and the linker between them must be rigid and restricted between 8 and 16 A. Accordingly, modifications that do not influence these parameters could improve the ability of curcumin against Abeta peptides. In 2008, Narlawar et al. replaced the 1,3-dicarbonyl moiety with isosteric heterocycles to minimize the metal chelation properties of curcumin and to lock its conformation into an enol-type arrangement. This modification provided potent ligands of Abeta aggregates and inhibitors of tau protein aggregation and depolymerized tau protein aggregates at low micromolar concentrations.

As a matter of fact, the main limitations of natural curcumin is its total lack of solubility in physiological conditions and its high instability.

Current strategies include development of curcumin analogues with similar biological activity but improved pharmacokinetics, bioavailability, water solubility and stability at physiological conditions. Another possibility for the application of the beneficial effects of curcumin could be the attachment of curcumin, in a stable way and active structural conformation, on the surface of biocompatible/biodegradable and stealth nanoparticles. Such attachment (on the surface of nanoparticles), might help increase the drug bioavailability and perhaps it will further increase the binding affinity of curcumin for Aβ peptides, due to multivalency, as well.

Among the known nanoparticle types, liposomes have many advantages for drug delivery applications due to their non-toxic and non-immunogenic, fully biodegradable and structurally versatile nature.

There exists the problem nowadays that up-to-date there is the need for curcumin-derivative with the properties required for such an application as described above, namely with improved stability and improved solubility compared to curcumin. Furthermore, there is the need for realization of a nanoparticulate system decorated with curcumin, or a derivative of curcumin, which may have high stability and high affinity for Aβ1-42 fibrils, in order for it to be useful for realizing the goals of the present invention.

With the present invention, we solve the above problem in that we have designed and synthesized new curcumin-derivatives based on the information mentioned above. We synthesized a series of compounds replacing the 1,3-dicarbonyl moiety with differently functionalized isosteric pyrazole rings, in order to guarantee the coplanarity and to improve solubility and stability in physiological conditions. Additionally, we formulated with two different methodologies nanosized liposomes functionalized with the curcumin derivatives on their surface. Their ability to bind to Aβ fibrils was evaluated by Surface Plasmon Resonance (SPR). The preparation of decorated liposomes could be carried out either by a conventional liposome preparative method (using a phospholipid conjugate of the curcumin derivative) or by a click chemistry technique, in which case the curcumin derivative is produced in situ on the liposomes, which are initially functionalized with an azide-phospholipid, by conjugation of an appropriate precursor of the curcumin derivative opportunely functionalized for the conjugation.

Both types of generated curcumin-decorated vesicles have mean diameters in the nano range (131-207 nm), which can be further reduced by extrusion if required, and are stable for periods up to 20 days, at least. They also demonstrate high integrity during incubation in presence of plasma proteins.

### SUMMARY OF THE INVENTION

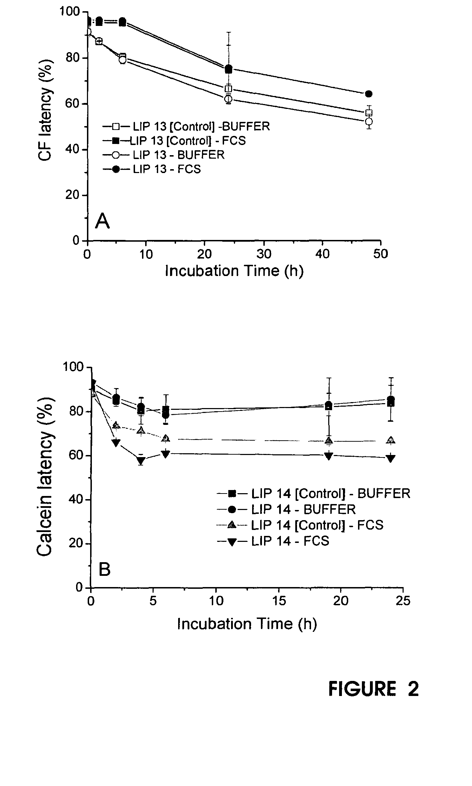

In the present invention, curcumin-derivatives with improved physicochemical properties and Curcumin-derivative-decorated liposomes are prepared, which are useful and solving existing problem that the invention comes to solve, due to their integrity, stability and binding affinity to Aβ1-42 fibrils. For preparation of curcumin-derivative- decorated vesicles (**13**), a click chemistry method was used, with use of an appropriate curcumin derivative. Initial vesicle integrity experiments revealed that the click chemistry reaction should be carried out at room temperature (Fig 2.B), since at 37 °C the liposome integrity is highly affected (Fig 2.A) by the reaction media required for the click to occur. Of course this is the case for the specific liposomes (with lipid composition of DPPC/DPPG/Chol 8:2:5 mol/mol/mol) evaluated herein; perhaps other lipid compositions may be more or less stable, in which case perhaps it would be required to use milder conditions for click attachment, in order to preserve vesicle integrity during the reaction to decorate their surface. For construction of liposomes a second method may be also used for attachment of the curcumin-derivative on the surface of vesicles, after performing the click reaction between compounds in which case the planar structure of curcumin, required for its activity [34], is disrupted.

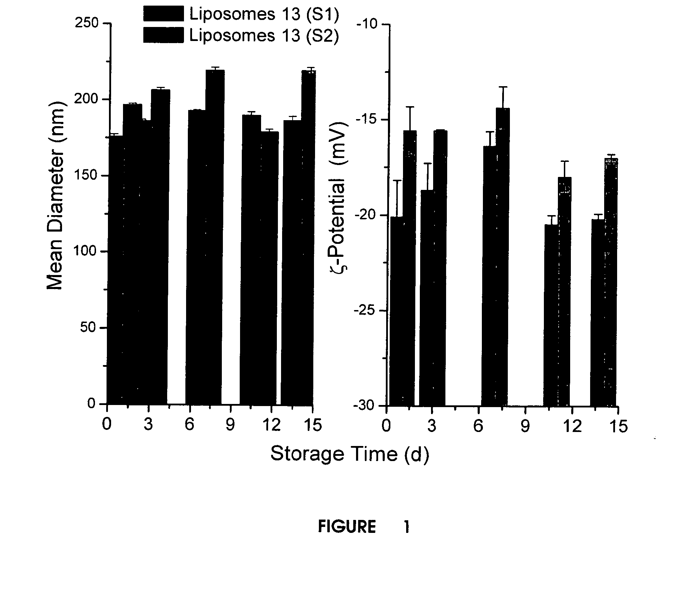

Both methods utilized herein for formation of curcumin-decorated nanosized liposomes are successful to prepare nanosized liposomes (Table 1) with the appropriate stability to be used for in vivo applications (Fig. 1 & 2) and high stability during storage (Fig. 1). As the amount of curcumin molecules attached to the vesicle surface increases, the vesicles produced (by both techniques evaluated) demonstrated an increase in mean diameter, as anticipated due to the curcumin coating, however the vesicle population was still homogeneous, as judged by the low polydisperity indices of the dispersions (Table 1).

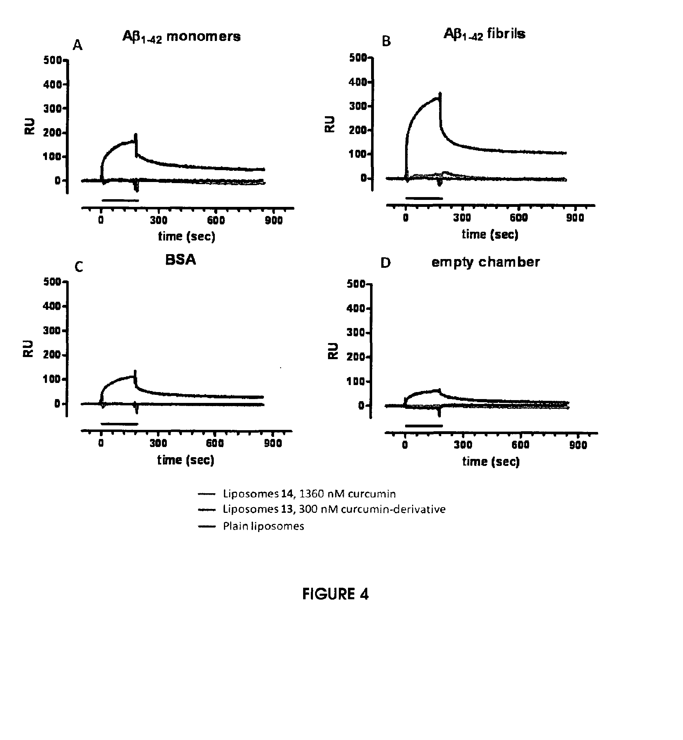

SPR binding results (Fig. 4) show that the plain liposomes (**18**) not-decorated with curcumin, do not show any binding affinity to the immobilized Aβ1-42 fibrils; whereas the vesicles exposing a curcumin derivative maintaining the planarity showed very high binding. This result proves that this specific structural characteristic is indeed required for curcumin binding to the fibrils.

Surpisingly, the affinity of the curcumin derivative - exposed on liposomes 18 - for Abeta fibrils (2-10 nM) is much higher than the affinity of a corresponding compound not attached to liposomes (7 uM). We suggest the involvement of multivalent interactions, i.e. different molecules of curcumin derivative on the same liposome contribute to the binding to the immobilized Aβ1-42 fibrils. It has been previously shown, in fact, that a multivalent ligand, dendrimer ; or nanoparticle, has a binding affinity for its target which can greatly exceed, even by 2-3 orders of magnitude, the binding affinity of the same ligand, if monovalent. This increase of affinity is due, in particular, to a decrease of the dissociation rate constants, approaching those of a pseudo-irreversible binding, and the same finding is actually found with the liposomes described in the present invention, decorated with the curcumin derivatives. The binding of the decorated liposomes for BSA was much lower and this is consistent with the lack of binding of the curcumin derivative for this plasmatic protein.

Advantageously, the click chemistry methodology is successfully utilized in the present invention for decoration of the surface of nanoliposomes with a curcumin derivative which retain the structural characteristics required for the antifibrillogenic activity. These nanosized curcumin-decorated liposomes showed the highest affinity for Aβ1-42 fibrils (1-5 nM) reported up-to-date, and sufficient integrity/stability for in vivo applications. Thus, they are potentially very useful in the attempt to target these AD pathogenic markers for diagnostic and/or therapeutic purposes.

The following compounds may be advantageously used, as they are or as basis to form derivatieves thereof for the decoration of the liposomes:
Preferably compounds of the general formula (I) and (II) are used

| | |
|---|---|
| 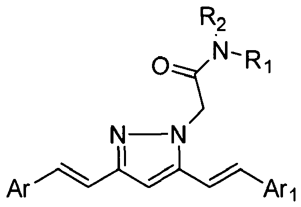 | 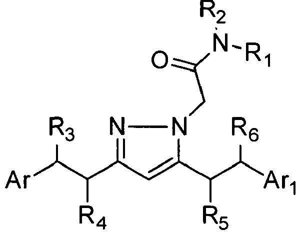 |
| I | II |

wherein R1-R2, equal or different can be hydrogen, alkyl chain, alkenyl chain, alkinyl chain, polymeric chain, cycloalkyl, cycloalkenyl, cycloalkinyl, aryl, heteroaryl or -OH; and Ar-Ar1, equal or different can be 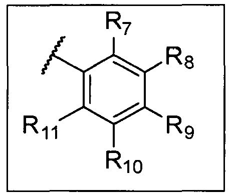 wherein R7, R8, R9, R10 and R11, equal or different can be hydrogen, halogens, alkyl chain, alkenyl chain, alkinyl chain, polymeric chain, cycloalkyl, cycloalkenyl, cycloalkinyl, aryl, heteroaryl, -SR12, -SSR13, -OR14, -NR15R16, where R12, R13, R14, R15, R16 equal or different, represent hydrogen, alkyl chain, alkenyl chain, alkinyl chain, polymeric chain, cycloalkyl, cycloalkenyl, cycloalkinyl, aryl, heteroaryl or -C(X)R17 (except - SSR13) where X could be O, N, S, and R17 represents hydrogen, alkyl chain, alkenyl chain, alkinyl chain, polymeric chain, cycloalkyl, cycloalkenyl, cycloalkinyl, aryl, heteroaryl, -SR18, -OR19, -NR20R21, where R18, R19, R20, R21 equal or different, represent hydrogen alkyl chain, alkenyl chain, alkinyl chain, polymeric chain, cycloalkyl, cycloalkenyl, cycloalkinyl, aryl, heteroaryl.

Advantageously can also be used compounds of general formula (II) wherein R3, R4, R5, R6, equal or different can be hydrogen, halogens, alkyl chain, alkenyl chain, alkinyl chain, polymeric chain, cycloalkyl, cycloalkenyl, cycloalkinyl, aryl, heteroaryl, - SR22, -SSR23, -OR24, -NR25R26, where R22, R23, R24, R25, R26 equal or different, represent hydrogen, alkyl chain, alkenyl chain, alkinyl chain, polymeric chain, cycloalkyl, cycloalkenyl, cycloalkinyl, aryl, heteroaryl or -C(Y)R27 (except -SSR23) where Y could be O, N, S, and R27 represents hydrogen, alkyl chain, alkenyl chain, alkinyl chain, polymeric chain, cycloalkyl, cycloalkenyl, cycloalkinyl, aryl, heteroaryl -SR28, -OR29, - NR30R31, where R28, R29, R30, R31 equal or different, represent hydrogen, alkyl chain, alkenyl chain, alkinyl chain, polymeric chain, cycloalkyl, cycloalkenyl, cycloalkinyl, aryl, heteroaryl.

Advantageously may also be used compounds of the general formula (I) and (II) wherein R1 or R2 equal or different could be

| | |
|---|---|
| 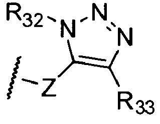 | 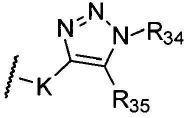 |

Wherein R32, R33, R34, R35 equal or different can be hydrogen, alkyl chain, alkenyl chain, alkinyl chain, polymeric chain, cycloalkyl, cycloalkenyl, cycloalkinyl, aryl, heteroaryl; Z, K can be alkyl chain, alkenyl chain, polymeric chain, cycloalkyl, cycloalkenyl, aryl or heteroaryl.

Preferably are used compounds of formula (I) according to claim 1 wherein R1 = propargyl, R2 = H, Ar=Ar1= 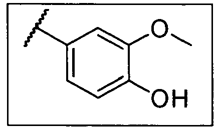 which is: 2-(3,5-bis((E)-4-hydroxy-3-methoxystyryl)-1H-pyrazol-1-yl)-N-(prop-2-yn-1-yl)acetamide

Advantageously are used compounds of formula (I) according to claim 1 wherein R1=H,R2=

| | | |
|---|---|---|
| 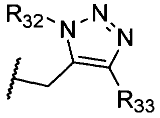 | or | 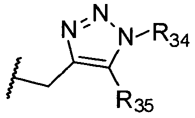 |

Wherein R33/R35 = H and R32/R34 = -CH₂CH₂OCH₂CH₂OCH₂CH₂OH and Ar=Ar1=

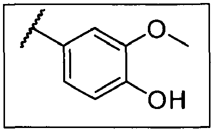

Which is:
2-(3,5-bis((E)-4-hydroxy-3-methoxystyryl)-1H-pyrazol-1-yl)-N-((1-(2-(2-(2-hydroxyethoxy)ethoxy)ethyl)-1H-1,2,3-triazol-4-yl)methyl)acetamide

### FIGURES

**FIGURE 1** shows the size (d-hydrodynamic diameter, nm) and ζ-Potential values of two different vesicle batches of liposomes (decorated with 5mol% [S1] or 10mol% [S2] curcumin), dispersed in PBS buffer at a lipid concentration of 10 - 15 mg/ml, during storage at 4 °C. Each value is the mean of at least 5 different measurements and bars are the SD values of each mean
**FIGURE 2** shows the integrity of cucrumin-derivative decorated liposomes and their corresponding control liposomes (with no curcumin on their surface), during incubation in buffer or FCS (80% v/v) at 37 °C. Each data point is the mean of at least 3 different experiments and the bar is the SD of the mean.

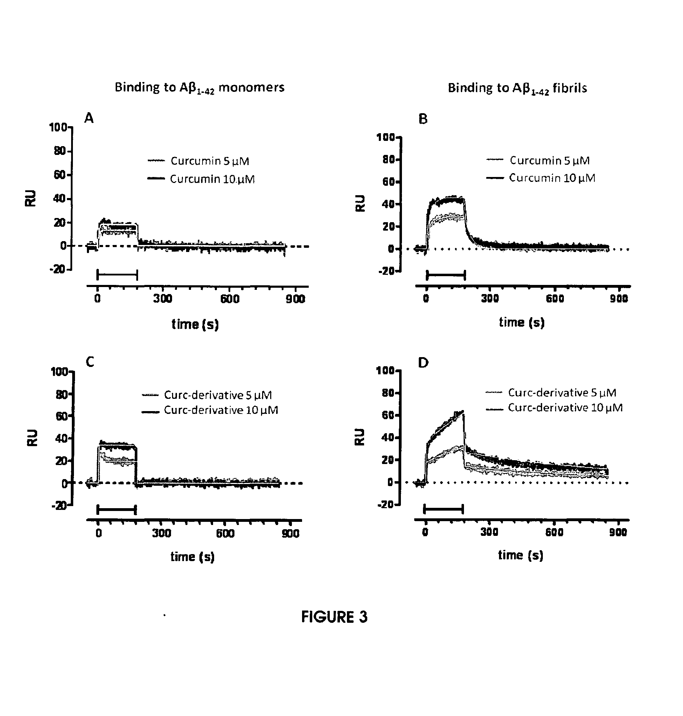

**FIGURE 3** shows SPR studies comparing the binding properties of curcumin and its derivative for Aβ₁₋₄₂ monomers or fibrils, immobilized on the sensor chip. The figure shows representative sensorgrams (resonance units, RU, *versus* time) obtained by simultaneous injection of two concentrations of the compounds, for 3 min (as indicated) over sensor chip surfaces. The reported sensorgrams are indicative of a specific binding to Aβ₁₋₄₂, since they were obtained after subtraction of the signal detected in the reference surface, thus correcting for binding-independent responses, such as bulk effects due to buffer exchanges or drift effects. The fitting of these sensorgrams, with a simple "one-site" model (for the monomers) or with a "two-sites" model (for fibrils) are shown in *white.*
**FIGURE 4** shows SPR studies comparing the binding properties of liposomes **18** and plain liposomes when injected onto sensor surfaces immobilizing Aβ1-42 monomers (A), Aβ1-42 fibrils (B) or bovine serum albumine (BSA) (C), at similar densities; panel D refers to the signal measured in the reference, empty, surface. Liposomes 18 (red) were injected at a concentration corresponding to 300 nM of exposed curcumin-derivative; plain liposomes 14 (blue) were injected at a concentration corresponding to 1360 nM of exposed curcumin.

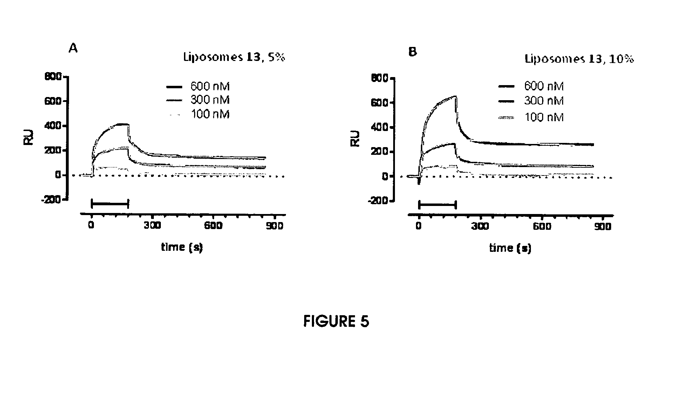

**FIGURE 5** shows the concentration-dependent binding of liposomes **18** to Aβ1-42 fibrils immobilized on the SPR sensor chip. For this session, liposomes with two different densities of functionalization with the curcumin-derivative (5 and 10%) were used. Liposomes were injected at concentrations corresponding to 100, 300 and 600 nM of exposed curcumin-derivative. The figure shows representative sensorgrams (resonance units, RU, versus time) obtained by simultaneous injection of the liposomes, for 3 min over sensor chip surfaces (as indicated). The reported sensorgrams are indicative of a specific binding to Aβ1-42, since they were obtained after subtraction of the signal detected in the reference surface, thus correcting for binding-independent responses, such as bulk effects due to buffer exchanges or drift effects. The fitting of these sensorgrams, with a "two-sites" model, are shown in white.

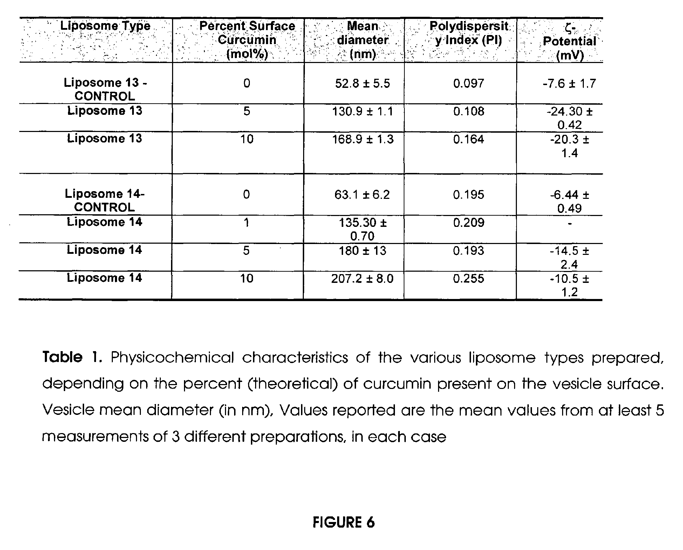

**FIGURE 6** shows Table 1, where are seen the physicochemical characteristics of the various liposome types prepared, depending on the percent (theoretical) of curcumin present on the vesicle surface. Vesicle mean diameter (in nm), Values reported are the mean values from at least 5 measurements of 3 different preparations, in each case

### 2. MATERIALS AND METHODS

### 2.1. Materials

### For lipid conjugate synthesis:

All commercial chemicals were purchased from Sigma-Aldrich except O-(2-azidoethyl)-O'-(N-diglycolyl-2-aminoethyl)heptaethyleneglycol which was purchased from Novabiochem and 1,2-Dipalmitoyl-*sn*-Glycero-3-Phosphothioethanol (Sodium Salt) which was obtained from Avanti polar lipids. All chemicals were used without further purification, while all required anhydrous solvents were dried with molecular sieves, for at least 24 h prior to use. Thin layer chromatography (TLC) was performed on silica gel 60 F254 plates (Merck) with detection using UV light when possible, or by charring with a solution of (NH₄)₆Mo₇O₂₄ (21 g), Ce(SO₄)₂ (1 g), concentrated H₂SO₄ (31 mL) in water (500 mL) or with an ethanol solution of ninhydrin or with Dragendorff spray reagent or molybdenum blue spray. Flash column chromatography was performed on silica gel 230-400 mesh (Merck). ¹H and ¹³C NMR spectra were recorded at 25°C unless otherwise stated, with a Varian Mercury 400 MHz instrument. Chemical shift assignments, reported in ppm, are referenced to the corresponding solvent peaks. MS were recorded either on a QTRAP system with ESI source (Applied Biosystem) while HRMS were registered on a QSTAR elite system with a nanospray ion source (Applied Biosystem).

### For nanoliposome preparation:

Phosphaditidyl choline (PC), 1,2-distearoyl-sn-glycerol-3-phosphatidylcholine (DSPC), 1,2-dipalmitoyl-sn-glycerol-3-phosphatidylcholine (DPPC), Phosphatidyl glycerol (PG), were purchased from Avanti Polar Lipids. Cholesterol (Chol), Sephadex G50 (course,) 5,6 carboxyflouorescein (CF), calcein, Sepharose CL-4B were purchased from Sigma- Aldrich. Spectrapore Dialysis tubing (MW cutoff 10000) was from Serva. Other phospholipids can also be used , for modulation of liposome properties.

All other chemicals were reagent grade. Ultrapure water was produced with a Millipore Direct-Q system.

### 2.2 Synthesis of curcumin derivatives-Chemical Synthesis procedures

### Synthetic Scheme 1:

| |
|---|
| 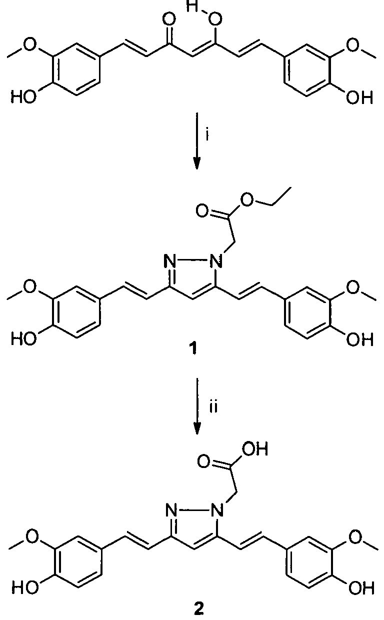 |
| *Reagents and Conditions:* (i) ethyl 2-hydrazinoacetate hydrochloride, TEA, toluene, reflux, 2.5 h; (ii) LiAlH₄, THF dry, 0° to RT, 70 %; (iii) KOH 1.8 M methanolic solution, RT, O.N., 87 % over two steps. |

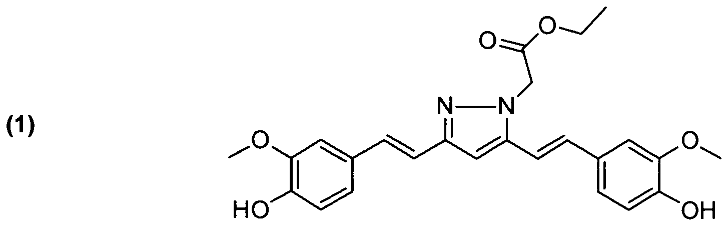

### Ethyl 2-(3,5-bis((E)-4-hydroxy-3-methoxystyryl)-1H-pyrazol-1-yl)acetate (1):

1. To a solution of Curcumin (1 g, 2.7 mmol, 1 eq) in Toluene dry (28 mL) was added ethyl 2-hydrazinylacetate hydrochloride (0.839 g, 5.4 mmol, 2 eq), triethylamine (0.753 mL, 5.4 mmol, 2 eq), TFA (0.1 mL, 1.3 mmol, 0.5 eq) and held at reflux for 2.5 hours.
2. The reaction was monitored by TLC. The reaction mixture was then cooled to ambient temperature, diluited with CH₂Cl₂ and washed with HCl 5%_{(aq)} twice.
3. The organic phase was dried with Na₂SO₄ and evaporated in vacuo to afford the crude product which was further purified by flash column chromatographic (CH₂Cl₂/isopropanol 98/2) to afford the desired product as yellow solid (1.058 g, 87%).
4. ¹H NMR (400 MHz, DMSO *d₆*) δ ppm 7.22 - 6.70 (m, 11H, *CH* Ar and conjugated double bond), 5.16 (s, 2H *CH*₂C=O), 4.13 (q, *J* = 7.05 Hz, 2H, O*CH*₂CH₃), 3.82 - 3.77 (m, 6H, OC*H₃*), 1.19 (t, *J* = 7.12 Hz, 3H, OCH₂*CH₃*). ¹³C NMR (100 MHz, DMSO *d₆*) δ 168.32 (C=O), 150.02, 147.86, 147.27, 146.68, 143.35 (5 CqAr), 132.25, 129.79 (2 CHAr), 128.49, 128.06 (2 CqAr), 120.81, 120.01, 117.75, 115.57, 111.66, 110.24, 109.58, 98.63 (8 CHAr and conjugated double bond), 61.11 (CH₂), 55.76, 55.59 (2 CH₃), 50.30 (CH₂), 14.09 (CH₃). MS (ESI) calcd for [M + H]⁺ 451.48; found [M + H]⁺ 451.60. Elemental analysis calcd (%) for C₂₅H₂₆N₂O₆: C 66.65, H 5.82, N 6.22; found: C 66.39, H 5.80, N 6.20

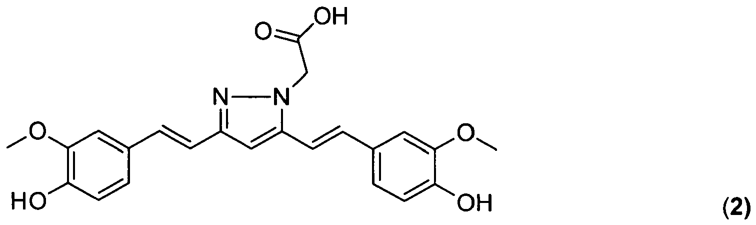

### 2-(3,5-bis((E)-4-hydroxy-3-methoxystyryl)-1H-pyrazol-1-yl)acetic acid (2):

**1.** Compound **1** (3g, 6,66 mmol, 1 eq) was dissolved in a methanolic solution of KOH 1.8M (70 ml). The solution was left under magnetic stirring at r. t. over night. The reaction was monitored by TLC.
2. The solvent was evaporated in vacuo and purified by flash column chromatographic (eluent ethyl acetate/methanol gradient from 9/1 to 7/3) to afford the final product (2.813 g, quant.).
3. ¹H NMR (400 MHz, CD₃OD) δ ppm 7.23 - 6.72 (m, 11H, C*H* Ar and conjugated double bond), 4.93 (s, 2H, C*H*₂C=O), 3.85 - 3.79 (m, 6H, OC*H*₃). ¹³C NMR (100 MHz, DMSO) δ 170.17 (C=O), 147.85, 147.16, 146.58, 142.80 (4 CqAr), 131.54, 129.13 (2 CHAr), 128.62, 128.17 (2 CqAr), 120.61, 119.87, 118.08, 115.58, 112.31, 110.08, 109.53, 98.38 (8 CHAr and conjugated double bond), 55.74, 55.58 (2 CH₃), 51.58 (CH₂). MS (ESI) calcd for [M + H]⁺ 423.43; found [M + H]⁺ 423.4. Elemental analysis calcd (%) for C₂₃H₂₂N₂O₆: C 65.38, H 5.25, N 6.63; found: C 65.18, H 5.23, N 6.62.

### Synthetic Scheme 2:

| |
|---|
| 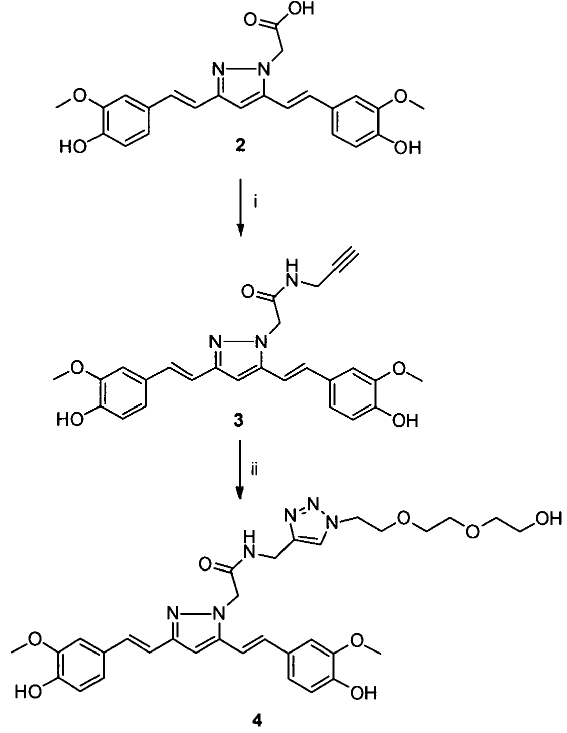 |
| *Reagents and Conditions:* (i) Propargyl amine, TBTU, HoBT, TEA, DMF dry, RT, O.N., |
| 63 %; (ii) 2-(2-(2-azidoethoxy)ethoxy)ethanol, Cu(I), THF/H₂O, RT, O.N., 72 % |

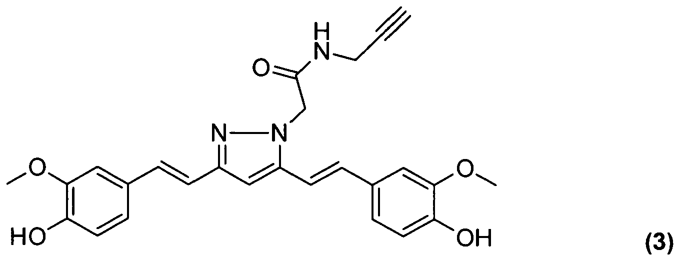

### 2-(3,5-bis((E)-4-hydroxy-3-methoxystyryl)-1H-pyrazol-1-yl)-N-(prop-2-yn-1-yl)acetamide (3):

1. Hobt (0.017 g, 0.123 mmmol, 1.23 eq) and TBTU (0.039 g, 0.12 mmol, 1.2 eq) were added to a solution of the compound **2** (0.042 g, 0.1 mmol, 1 eq) in DMF dry (1 mL).
2. The reaction mixture was left in the dark, at r.t., under magnetic stirring for 15 minutes.
3. Propargylamin (0.013mL, 0.2 mmol, 2 eq) and triethylamine (0.07 mL, 0.5 mmol, 5 eq) were added and the reaction was left in the dark, at r.t., under magnetic stirring over night. T
4. The reaction was monitored by TLC. The solvent was evaporated in vacuo. The crude product was diluited with CH₂Cl₂ and washed three times with water.
5. The organic phase was dried with Na₂SO₄ and evaporated in vacuo to afford the crude product which was further purified by flash column chromatographic (eluent petroleum ether/ethyl acetate gradient from 3/7 to ethyl acetate) to afford the desired product as a powder (0.031 g, 68%).
6. ¹H NMR (400 MHz, CD₃OD) δ ppm 7.38 - 6.73 (m, 11H, C*H* Ar and conjugated double bond), 5.12 (s, 2H C*H*₂C=O), 4.04 (s, 2H, C*H*₂NH), 3.96 - 3.83 (m, 6H, OC*H*₃), 2.63 (s, 1H, C*H*). ¹³C (100 MHz, CDCl₃) δ ppm 169.31 (C=O) 152.55,149.33,148.03, 145.63 (4Cq Ar), 135.08, 132.22 (2 CHAr), 130.75, 130.13 (2 CqAr), 122.07, 121.40, 118.27, 116.46, 112.40, 111.08, 110.70, 100.52 (8 CHAr and conjugated double bond),80.27 (Cq), 72.42 (CH), 56.64, 56.55(2 CH₃), 52.66 (CH₂), 29.71(CH₂). MS (ESI) calcd for [M + H]⁺ 456.49; found [M + H]⁺ 460.4. Elemental analysis calcd (%) for C₂₆H₂₅N₃O₅: C 67.96, H 5.48, N 9.14; found: C 67.72, H 5.47, N 9.11.

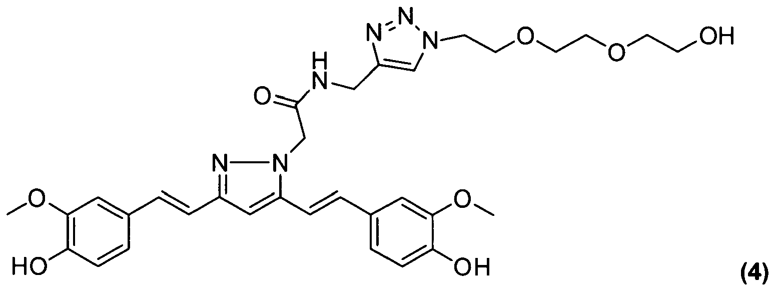

### 2-(3,5-bis((E)-4-hydroxy-3-methoxystyryl)-1H-pyrazol-1-yl)-N-((1-(2-(2-(2-hydroxyethoxy)ethoxy)ethyl)-1H-1,2,3-triazol-4-yl)methyl)acetamide (4):

1. CuSO₄*5H₂O (0.057 mg, 0.228 mmol, 1.2 eq) was dissolved in water (2 mL) and sodium ascorbate (0.057 g, 0.285 mmol, 1.5 eq) was added..
2. The reaction was stirred until the formation of an orange suspension. This freshly prepared catalyst was added to a solution of compond 3 (0.086 g, 0.19 mmol, 1 eq) and 2-(2-(2-azidoethoxy)ethoxy)ethanol **8** (0.252 g, 0.91 mmol, 1.2 eq) in THF (12 mL), stirring at r.t. until the formation of a light blue precipitate, which was removed by filtration.
3. The crude product was purified by flash column chromatographic (eluent gradient from ethyl acetate to ethyl acetate/methanol 9/1) to afford the desired product (0.103 g, 72%).
4. ¹H NMR (400 MHz, CD₃OD) δ ppm 7.83 (s, 1H, *H* triazole), 7.16-6.72 (m, 11H C*H* Ar and conjugated double bond), 4.97 (s, 2H, C*H*₂C=O), 4.47 (s, 2H, NH*CH*₂), 4.38 (t, *J* = 4.99 Hz, 2H, N*CH*₂CH₂O), 3.91 - 3.86 (m, 6H, OC*H*₃), 3.75 (t, *J* = 4.93, 2H, NCH₂*CH*₂O), 3.64 - 3.57 (m, 2H, OCH₂C*H*₂OH), 3.55 - 3.49 (m, 4H, OC*H*₂CH₂OH, OC*H*₂C*H*₂O), 3.48 - 3.43 (m, 2H, C*H*₂O).¹³ C NMR (100 MHz, MeOD) δ 169.65 (C=O), 152.46, 149.30, 149.23, 147.98, 145.81, 145.55 (6 CqAr), 134.92, 132.19 (2 CHAr), 130.59, 129.96 (2 CqAr), 125.11, 122.03, 121.35, 118.14, 116.36, 112.28, 110.71, 110.35, 100.33 (9 CHAr and conjugated double bond), 73.62, 71.39, 71.34, 70.22, 62.20 (5 CH₂), 56.49, 56.39 (2 CH₃), 51.28, 35.78 (2 CH₂).MS (ESI) calcd for [M + H]⁺ 635.28; found [M + H]⁺ 635.5. Elemental analysis calcd (%) for C₃₂H₃₈N₆O₈: C 60.56, H 6.03, N 13.24; found: C 60.35, H 6.01, N 13.19.

### Synthetic Scheme 3:

| |
|---|
| 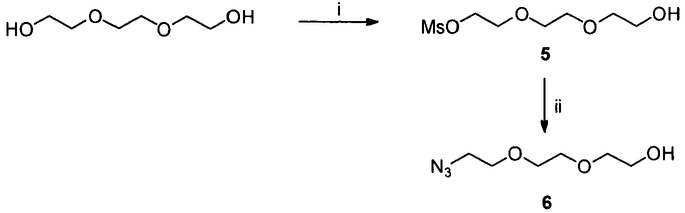 |
| *Reagents and Conditions:* (i) MsCl, Ag₂O, CH₂Cl₂, 36 h, R.T., 61 %; (ii) NaN₃, DMF, reflux, O.N., 80 %. |

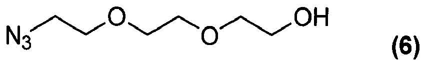

### 2-(2-(2-azidoethoxy)ethoxy)ethanol (6): the compound 6 was synthesized according to the procedure of Schneekloth et al.ⁱ.

### 2.3 Synthesis of lipid and curcumin-derivatives for liposome formation by click-chemistry

For the preparation of curcumin-decorated liposomes by the click reaction, two specific compounds which were specially synthesized were used,; a lipid-peg-azide compound (3-deoxy-1,2-dipalmitoyl-3-(4'-methyl (O-(2-azidoethyl)-heptaethylenglycol-2-yl)-ethylcarbamoylmethoxy ethylcarbamoyl-1H-1',2',3'-triazol-1'-yl)-*sn*-glycerol **11**), and a curcumin derivative with a terminal alkyne group (*N*-propargyl 2-(3',5'-di(4-hydroxy-3-metoxystyryl)-1H-pyrazol-1'-yl)-acetamide **15**), as presented in synthetic schemes 4 and 5, respectively. Details about the conditions and quantities used for the synthesis of all intermediate and final compounds are reported here.

### Synthetic Scheme 4:

| |
|---|
| 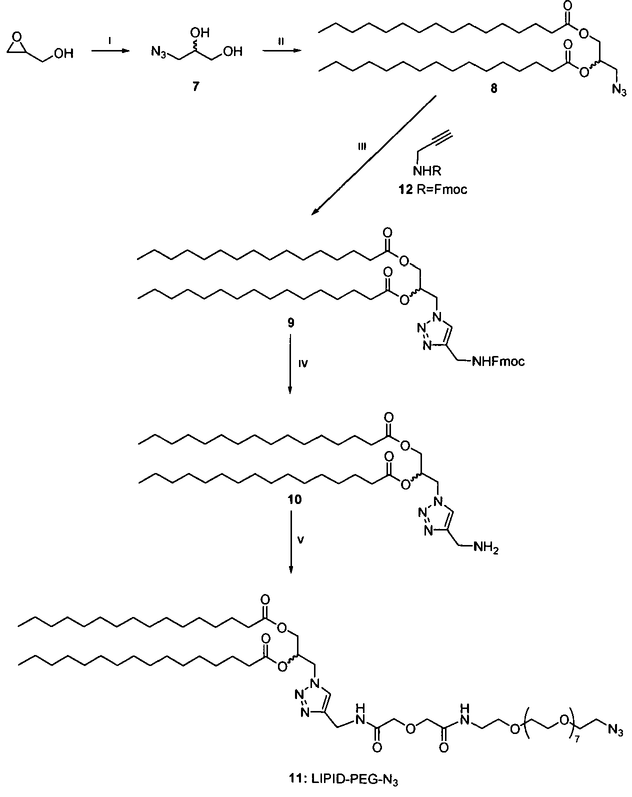 |
| *Reagents and Conditions:* (i) LiBF₄, NaN₃, *t*-BuOH/H₂O, reflux, 1 h; (ii) PalmitoylCl, Py, CH₂Cl₂ dry, RT, O.N., 75 % over two steps; (iii) Cu(I), THF/H₂O, RT, O.N., 87 %; (iv) Piperidine, DMF, RT, 2 h; (v) TBTU, O-(2-azidoethyl)-O'-(N-diglycolyl-2-aminoethyl) heptaethyleneglycol, DIPEA, CH₂Cl₂ dry, RT, 3 dd, 92 %. |

### Synthetic procedures:

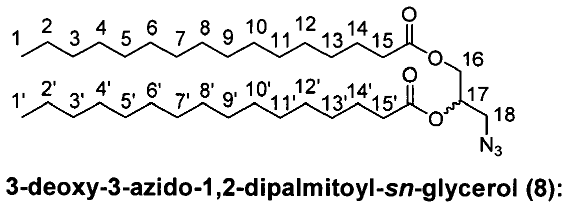

1. The 3-azido-1,2-diol-propane **7** is synthesized according to the procedure of Kazemi *et al*.. Lithium tetrafluoroborate (0.190 g, 2 mmol, 0.2 eq) and sodium azide (1.30 g, 20 mmol, 2 eq) are added to a solution of 2-oxiranylmethanol (0.660 mL, 10 mmol, 1 eq) in 30 mL of *t*-butanol/H₂O (30:1). The reaction mixture is stirred under reflux for 2 h. The solvent is removed and the crude product is purified by chromatography on short column of silica-gel to eliminate salts.
2. This purified azido alcohol, as a very unstable compound, is used in the next step without further characterization. Palmitoyl chloride (6.55 mL, 21.6 mmol, 2.4 eq) is added dropwise under argon to a solution of **7** (1.05 g, 9 mmol, 1 eq) and dry pyridine (2.9 mL, 36 mmol, 4 eq) in dry CH₂Cl₂ (50 mL). The resulting solution is stirred until disappearance of starting material.
3. The reaction mixture is diluted with CH₂Cl₂ and washed with an aqueous solution of HCl (5 %) and the aqueous layer was extracted with CH₂Cl₂ three times. The combined organic layer is dried with Na₂SO₄ and evaporated. The crude is purified by flash chromatography (eluent petroleum ether/AcOEt 99/1) to afford the title compound 8 (4.45 g, 7.5 mmol, 75 % over the two steps) as white wax.
4. ¹H NMR (400 MHz, CDCl₃) δ ppm 5.21 -5.14(m, 1H, 17), 4.29 (dd, *J* = 11.9, 4.4 Hz, 1H, 16a), 4.14 (dd, *J* = 11.9, 5.6 Hz, 1H, 16b), 3.46 (m, 2H, 18), 2.38 - 2.28 (m, 4H, 15,15'), 1.68 - 1.56 (m, 4H, 14,14'), 1.25 (bs, 48H, 2-13, 2'-13'), 0.88 (t, *J* = 6.6 Hz, 6H, 1,1'); ¹³C (100 MHz, CDCl₃) δ ppm 173.40, 173.03 (2 C=O), 70.08 (CH), 62.49, 51.07, 34.39, 34.24, 32.16, 29.93, 29.89, 29.85, 29.70, 29.60, 29.49, 29.33, 29.29, 25.08, 25.03, 22.92 (16 CH2), 14.34(CH3); MS (ESI) calcd for [M + H]⁺ 594.93; found [M + H]⁺ 594.89.

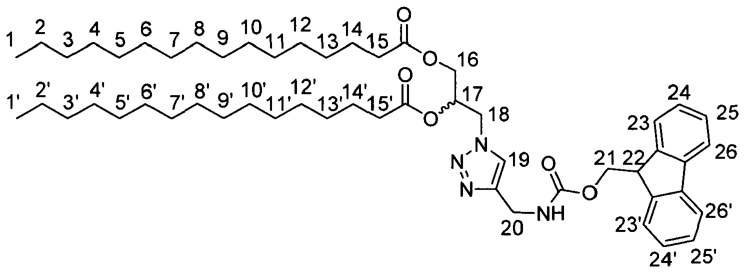

### 3-deoxy-1,2-dipalmitoyl-3-(4'-(9H-fluoren-9"-yl)methoxy methylcarbamoyl-1H-1',2',3'-triazol-1'-yl)-sn-glycerol (9):

1. CuSO₄·5 H₂O (0.227 g, 0.91 mmol, 1.2 eq) is dissolved in H₂O (4 mL) and sodium ascorbate (0.226 g, 1.14 mmol, 1.5 eq) is added. The solution is stirred until the formation of an orange suspension. This freshly prepared catalyst is added to a solution of compound **8** (0.450 g, 0.76 mmol, 1 eq) and compound **11** (0.252 g, 0.91 mmol, 1.2 eq) in THF (12 mL), stirring at R.T. until the formation of a light blue precipitate, which is removed by filtration.
2. The filtrate is diluted with ethyl acetate and washed with water three times. The combined organic layer is anhydrificated with Na₂SO₄ and evaporated under reduced pressure. The crude product is purified by flash chromatography (eluent petroleum ether/ethyl acetate gradient from 9/1 to 1/1) to afford the compound **9** (0.578 mg, 0.66 mmol, 87%) as light yellow oil.
3. ¹H NMR (400 MHz, CDCl₃) δ ppm 7.75 (d, *J* = 7.5 Hz, 2H, 26,26'), 7.60 - 7.54 (m, 3H, 19,23,23'), 7.39 (t, *J* = 7.4 Hz, 2H, 25,25'), 7.29 (t, *J* = 7.4 Hz, 2H, 24,24'), 5.55 - 5.48 (m, 1H, N*H*), 5.42 - 5.32 (m, 1H, 17), 4.63 - 4.52 (m, 2H, 18), 4.49 - 4.43 (m, 2H, 17), 4.41 - 4.35 (m, 2H, 21), 4.30 (dd, *J* = 12.1, 4.4 Hz, 1H, 16a), 4.24 - 4.13 (m, 1H, 22), 4.05 (dd, *J* = 12.1, 5.2 Hz, 1H, 16b), 2.37 - 2.24 (m, 4H, 15,15'), 1.66 - 1.48 (m, 4H, 14,14'), 1.36 - 1.17 (m, 48H, 2-13, 2'-13'), 0.87 (t, *J* = 6.8 Hz, 6H, 1,1'); ¹³C NMR (100 MHz, CDCl₃) δ ppm 173.37, 172.79, 156.62 (3 C=O), 145.46, 144.01, 141.51 (3 CqAr), 127.94, 127.27, 125.29, 123.24, 120.23 (5 CHAr), 69.51 (CH), 67.16, 62.19, 50.31 (3 CH₂), 47.35 (CH), 36.61, 34.24, 32.17, 29.95, 29.92, 29.88, 29.73, 29.62, 29.53, 29.50, 29.37, 29.27, 25.08, 24.97, 22.95 (15 CH₂), 14.40 (CH₃); MS calcd for [M + H]⁺ 872.27; found [M + H]⁺ 871.24.

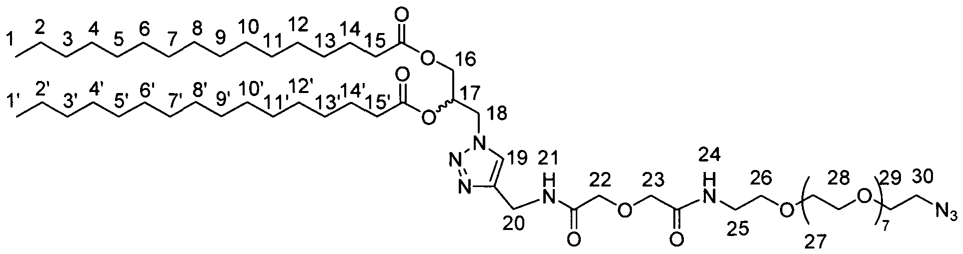

### 3-deoxy-1,2-dipalmitoyl-3-(4'-methyl (O-(2-azidoethyl)-heptaethylenglycol-2-yl)-ethylcarbamoylmethoxy ethylcarbamoyl-1H-1',2',3'-triazol-1'-yl)-sn-glycerol (11) [LIPID-PEG-N₃]:

1. Compound **9** (0.250 g, 0.29 mmol, 1.1 eq) is dissolved in dry DMF (3 mL) and piperidine (26 µL, 10 µL/0.11 mmol) is added. The reaction is stirred until the complete disappearance of the starting material. The reaction mixture is evaporated under high vacuum to afford the analytically pure compound **10.**
2. This compound is dissolved in CH₂Cl₂ (5 mL) and is added dropwise to a solution of O-(2-azidoethyl)-O'-(N-diglycolyl-2-aminoethyl)heptaethyleneglycol (0.145 g, 0.26 mmol, 1 eq), TBTU (0.109 g, 0.34 mmol, 1.3 eq) and DIPEA (60 µL, 0.34 mmol, 1.3 eq) in CH₂Cl₂ after 20 min of stirring. The reaction mixture is stirred for 3 days until the disappearance of the starting polyethyleneglycol compound.
3. The reaction solution is evaporated under reduced pressure and the crude product is purified by flash chromatography (eluent ehyl acetate/methanol 95/5) do afford the compound **11** (0.285 g, 0.24 mmol, 92 %), as white solid.
4. ¹H-NMR (400 MHz, CDCl₃) δ ppm 7.91 - 7.84 (m, 1H, 21), 7.60 (s, 1H, 19), 7.48 - 7.40 (m, 1H, 24), 5.34 - 5.27 (m, 1H, 17), 4.53 - 4.44 (m, 4H, 18,20), 4.25 (dd, *J* = 12.1, 4.0 Hz, 1H, 16a), 4.02 - 3.94 (m, 5H, 16b,22,23), 3.65 - 3.53 (m, 32H, 26,27,28,29), 3.47 - 3.38 (m, 2H, 25), 3.36 - 3.29 (m, 2H, 30), 2.30 - 2.19 (m, 4H, 15,15'), 1.60 - 1.45 (m, 4H, 14,14'), 1.30 - 1.13 (m, 48H, 2-13, 2'-13'), 0.81 (t, *J* = 6.6 Hz, 6H, 1,1'); ¹³C NMR (100 MHz, CDCl₃) δ ppm 173.28, 172.73, 169.15, 168.92 (4 C=O), 145.07 (CqAr), 123.82 (CHAr), 71.05, 70.96, 70.78, 70.61, 70.19, 69.94 (6 CH₂), 69.49 (CH), 62.16, 50.81, 50.24, 38.98, 34.36, 34.15, 32.10, 29.88, 29.84, 29.81, 29.67, 29.54, 29.46, 29.45, 29.30, 29.21, 25.01, 24.91, 22.87 (19 CH₂), 14.33 (CH₃); HRMS calcd for [M + Na]⁺ 1207.8139; found [M + Na]⁺ 1207.8204.

### Synthetic Scheme 5:

| |
|---|
| 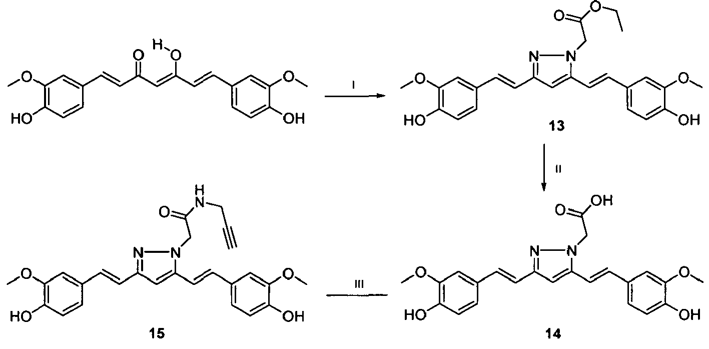 |
| *Reagents and Conditions:* (i) ethyl 2-hydrazinoacetate hydrochloride, TEA, toluene, reflux, O.N; (ii) KOH 1.8 M methanolic solution, RT, O.N., 87 % over two steps; (iii) Propargyl amine, TBTU, HOBT, TEA, DMF dry, RT, O.N., 63 %. |

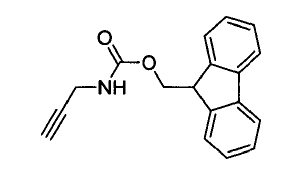

### (9H-fluoren-9-yl)methyl prop-2-yn-1-ylcarbamate (12):

1. Commercial propargylamine (0.277 mL, 4.34 mmol, 1 eq) is dissolved in dioxane (17 mL) and a 0.8 M aqueous solution of Na₂CO₃ (14 mL) was added. A solution of FmocCl (1.343 g, 5.2 mmol, 1.2 eq) in dioxane (17 mL) is freshly prepared and added dropwise to the amine solution.
2. The reaction is stirred for 1 h, until the disappearance of the starting material. The mixture is diluted with ethyl acetate and washed with H₂O three times. The organic layer is dried with Na₂SO₄ and concentrated. The crude solid is recrystallized from EtOAc to afford compound **12** (0.780 g, 2.81 mmol, 65 %) as white powder.
3. The ¹H-NMR and ¹³C-NMR agree with the characterization reported by Seth Horne *et al*. [2].

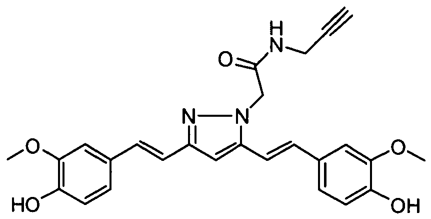

### N-propargyl 2-(3',5'-di(4-hydroxy-3-metoxystyryl)-1H-pyrazol-1'-yl)-acetamide (15):

The compound **15** was synthesized according to the procedure of La Ferla *et al.*

Compound **16,** formed by click reaction between lipid-peg-azide **11** and curcumin-alkyne derivative **15** in organic solvents is synthesized according to scheme 6. This new compound can be used for the preparation of curcumin-derivative decorated liposomes by conventional liposome preparation techniques.

### Synthetic Scheme 6:

| |
|---|
| 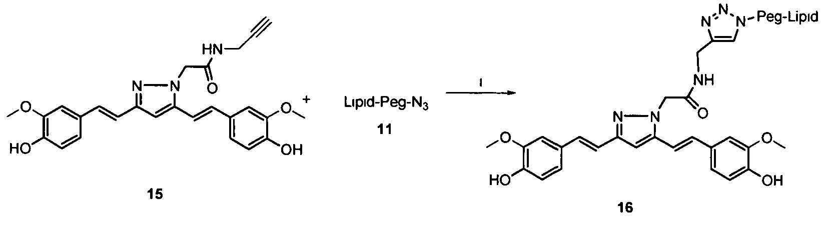 |
| *Reagents and Conditions:* (i) Procedure A: CuSO₄·5 H₂O, sodium ascorbate, THF-H₂O, RT, 87%; Procedure B: CuBr, PMDTA, sodium ascorbate, AcCN-H₂O, RT, 10 min, 92%. |

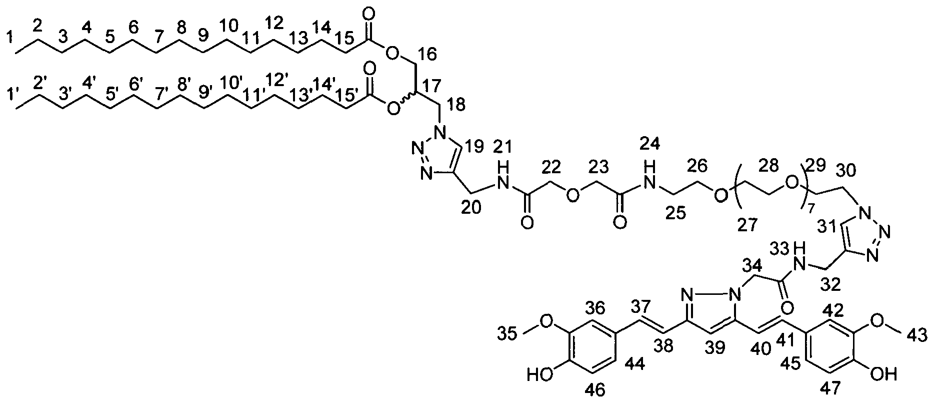

### 1,2-dipalmitoyl-3-(4'-(O-(2-(4"-(2-(3'",5"'-di(4-hydroxy-3-metoxystyryl)-1H-pyrazol-1"'-yl)-ethylcarbamoyl)-methyl-1H-1",2",3"-triazol-1"-yl-ethyl)-heptaethylenglycol-2-yl)-ethylcarbamoylmethoxyethylcarbamoyl-methyl-1H-1',2',3'-triazol-1'-yl)-sn-glycerol (16):

For this synthesis of 16 two different procedures may be followed:

### Procedure A:

1. CuSO₄·5 H₂O (0.007 g, 27.5 µmol, 1.2 eq) is dissolved in H₂O (0.6 mL) and sodium ascorbate (0.007 g, 34.4 µmol, 1.5 eq) is added. The solution is stirred until the formation of an orange suspension.
2. This freshly prepared catalyst is added to a solution of compound **11** (0.027 g, 23 µmol, 1 eq) and compound **15** (0.013 g, 27.5 µmol, 1.2 eq) in THF (1.8 mL). The mixture is stirred at R.T. until the formation of a light blue precipitate, which is removed by filtration.
3. The filtrate is evaporated under reduced pressure. The crude product is purified by flash chromatography (eluent ethyl acetate /methanol 85/15) to afford the compound **16** (0.025 g, 15.2 µmol, 87%) as brown wax.
4. ¹H-NMR (400 MHz, CDCl₃) δ ppm 8.03 - 7.91 (m, 1H, 21), 7.75 - 7.60 (m, 2H, 19,31), 7.50 - 7.43 (m, 1H. 24), 7.19 - 7.06 (m, 1H, 33), 7.08 - 6.81 (m, 9H, 36,37,39,41,42,44,45,46,47), 6.73 - 6.61 (m, 2H, 38,40), 5.41 - 5.30 (m, 1H, 17), 4.91 (s, 2H, 34), 4.60 - 4.46 (m, 6H, 20,32,18), 4.41 - 4.33 (m, 2H, 30), 4.29 (dd, J = 11.5, 3.5 Hz, 1H, 16a), 4.06 - 3.97 (m, 5H, 16b,22,23), 3.96 - 3.84 (m, 6H, 35,43), 3.71 - 3.79 (m, 2H, 29), 3.65 - 3.50 (m, 30H, 28,27,28), 3.49 - 3.43 (m, 2H, 25), 3.42 - 3.34 (m, 2H, 30), 2.35 - 2.23 (m, 4H, 15,15'), 1.67 - 1.52 (m, 4H, 14,14'), 1.35 - 1.15 (m, 48H, 2-13,2'-13'), 0.86 (t, J = 6.2 Hz, 6H, 1,1'); ¹³C NMR (100 MHz, CDCl₃) δ ppm 173.41, 172.86, 169.32, 169.12, 167.84 (5 C=O), 147.33, 147.16, 146.98, 146.16 (4 CqAr), 134.21, 131.09 (2 CHAr), 129.62, 128.67 (2 CqAr), 121.31, 120.89, 117.87, 115.20, 114.94, 111.15, 108.93, 108.36 (8 CHAr and CH conjugated C=C), 70.97, 70.93, 70.60, 70.25, 69.96 (5 CH₂), 69.52 (CH), 69.45 (CH₂), 68.51 (CH), 68.23, 62.21 (2 CH₂), 56.29, 56.09 (2 CH), 50.40, 39.05, 35.15, 34.20, 32.14, 29.92, 29.88, 29.72, 29.59, 29.50, 29.35, 29.26, 25.05, 24.95, 22.92 (15 CH₂), 19.03, 14.37 (2 CH₃); MS calcd for [M + Na]⁺ 1666.99; found [M + Na]⁺ 1667.14.

### Procedure B:

1. Curcumin-alkyne derivative **15** (7 mg, 15.18 µmole, 1.2 eq), lipid-peg-N₃ **11** (15 mg, 12.65 µmole, 1 eq), CuBr (3.63 mg, 25.304 µmole, 2 eq) and PMDTA (21.13 µl, 101.22 µmole, 8 eq) are dissolved in 700 µl Acetonitrile (AcCN).
2. Sodium ascorbate in H₂O (10 mg, 50.608 µmole, 2 eq) is added and the reaction mixture was sealed and kept at room temperature. TLC and HPLC analysis of the reaction mixture showed that 10 min after sodium ascorbate addition **15** is completely reacted and **16** is selectively produced.
3. The reaction mixture is vortexed to remove a light blue precipitate formed during the reaction and the reaction mixture is subjected to purification. AcCN is removed under reduced pressure and the oily product was re-dissolved in CHCl₃. The chloroform phase is acidified with 5% aq. HCl and extracted three times with 0.05% aq. NaCl.
4. The organic phase is dried over Na₂SO₄ and is further purified using a 10-ml silica gel column using CHCl₃/MeOH (9:1) as eluent to afford **16** (19,15 mg, 11,64 µmole, 92%).
5. ESI-MS: M+H calc: 1645.01; M+H found: 1645.48. ¹H and ¹³C-NMR analysis had the same profile as in procedure A.

### 2.4. Nanoliposome preparation

Curcumin derivative decorated nanoliposomes can be prepared with the techniques described below incorporating or encapsulating fluorescent or other labels or bio-active substances or drugs.

### 2.4.1. Curumin-decorated liposomes by click chemistry

Liposomes functionalized with the curcumine derivative (**9**) by the click chemistry method were prepared as described in Synthetic Scheme 7.

### Synthetic Scheme 7:

| |
|---|
| 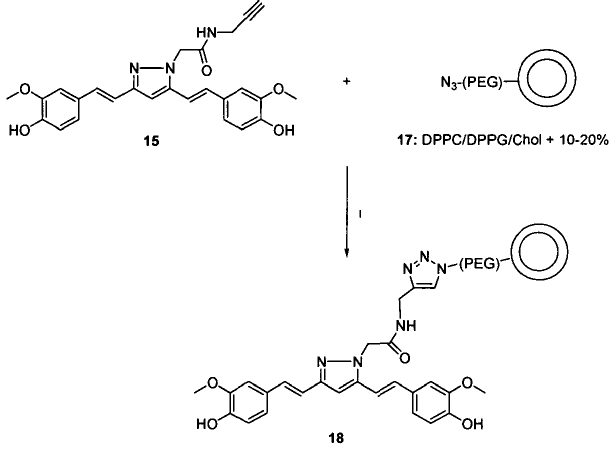 |
| *Reagents and Conditions:* (i) CuSO₄·5 H₂O, sodium ascorbate, bathophenanthrolinedisulfonate, buffer PBS 6,5 (No NaN₃), N₂, R.T., 6-8 hours. |

as follows in more detail:
1. SUV liposomes composed of DPPC/DDPG/Chol or other lipid compositions are initially prepared by any conventional liposome preparation technique (thin-film hydration, probe sonication, membrane extrusion, homogenization etc).
2. A dispersion of liposomes (**17**) is added in an aqueous solution of bathophenanthrolinedisulfonate catalyst (28 mM) mixed with a preformed aqueous solutions of CuSO₄ (8 mM) and sodium ascorbate (145 mM).
3. Then compound **15** (0,44mg solubilized in a small volume of DMSO) is added, and the reaction is gently stirred for 4-10 hours at 25-27°C under continuous flow of N₂.
4. Any aggregates that may have formed during the reaction are disaggregated by gentle bath sonication (∼ 2-5 min) and the resulting mixture is then purified
5. Purification is carried out by dialysis (placing the mixture in a 10.000 MW cutoff dialysis tubing (Servapore, Serva) and dialyzing against PBS buffer for at least 12 h. The vesicle dispersion may be further purified by column chromatography (Sephadex 4B).
6. Quantification of **16** in final liposomal dispersion **18** after liposomal click reaction may be achieved by HPLC analysis of a specific quantity of freeze-dried liposomal dispersion and integration of the corresponding peak. HPLC may be performed with a Lichrosphere 100 RP-18 (5µm column:); eluted with a CHCl₃/MeOH (9:1) + 0.08% TFA mobile phase; at a 1ml/min flow rate.

### 2.4.2. Curcumin-derivative decorated liposomes by conventional technique

For preparation of curcumine-decorated liposomes compound **16** is introduced in the lipid phase together with all the other lipids selected, in the initial step of liposome formation. Any liposomes preparation technique can be used.

### 2.5. Characterization of Nanoparticles

### 2.5.1. Size and polydispersity

The size and polydispersity of the liposome were determined using a NanoZeta series particle sizer; measurements were performed at 25 °C. (Malvern). Liposomes were found to be in the nano-dimension range (as seen in Table 1). In all cases, the polydispersity indices measured were low (ranging from 0.097 - 0.255) indicating that the nanoliposomes prepared have very narrow size distributions. For both of the liposome types prepared, vesicle stability was measured when the liposomes were dispersed in buffer (at various lipid concentrations) and stored at 4°C, by following their size, polydispersity index and ζ-potential (which were measured by dynamic laser light scattering as described above) for periods of 3 - 30 days. Bothe types were found to be stable, as demonstrated in Figure 1.

### 2.5.2. Liposome integrity

After carrying out the click chemistry reaction for decoration of the vesicle surface as described above, the vesicles produced were studied for their integrity during incubation in absence and in presence of serum proteins. For this, liposomes that encapsulated calcein (or CF), were prepared as described above and subjected to the click reaction for curcumin attachment to their surface. They were subsequently incubated in absence or presence of serum proteins (80% v/v, FCS) and their integrity was evaluated during their incubation at 37°C for 24 or 48 h. As seen in figure 2, the integrity of control liposomes (that have the same lipid composition but no curcumin on their surface) were also studied in parallel, while both types of liposomal dispersions (control and curcumin-decorated) were also incubated in presence of plain buffer for comparison. As demonstrated, both liposome types are equally stable when compared with the relevant control liposomes, during incubation in buffer, as well as in the presence of FCS, for a period of at least 24 h, indicating that these liposomes posses the required stability for in vivo applications.

In the case of curcumine-derivative decorated liposomes (mentioned as **13 in the graph**), the control liposomes were actually the same liposomes before the click reaction step was carried out. This result proves that under the specific conditions applied, liposomes which are pre-formed to encapsulate active drugs or other types of bioactive molecules can be subjected to click reaction and after appropriate purification, they can be used for in vivo applications.

### 2.6. Binding of liposomes to Aβ1-42, investigated by Surface Plasmon Resonance (SPR)

### 2.6.1. Preparation of Aβ1-42 in different aggregation forms

A depsi-Aβ1-42 peptide was synthetized at first, as previously described. This depsi□peptide is much more soluble than the native peptide and it also has a much lower propensity to aggregate, so preventing the spontaneous formation of 'seeds' in solution. The native Aβ1-42 peptide was then obtained from the depsi peptide by a "switching" procedure involving a change in pH. The Aβ1-42 peptide solution obtained immediately after switching is seed-free as shown in carefully conducted previous work. The Aβ1-42 peptide obtained with this procedure is therefore in its very initial state and, for sake of simplicity it will be referred to here as "monomers".

To prepare Aβ1-42 fibrils, the switched peptide solution was diluted with water to 100 µM, acidified to pH 2.0 with 1 M HCl, and left to incubate for 24 hrs at 37 °C [56]. Kinetic studies with circular dicroism and thioflavin-T clearly indicated that these conditions enable to reach the maximal level of β-sheet structures whereas the presence of amyloid fibrils was directly confirmed by atomic force microscopy (AFM).

### 2.6.2 Surface Plasmon Resonance (SPR) analysis

For these studies we used the ProteOn XPR36 (Biorad) apparatus, which has six parallel flow channels that can be used to uniformly immobilize strips of six "ligands" on the sensor surface. Aβ1-42 "monomers" or fibrils were immobilized in two of these parallel-flow channels of a GLC sensor chip (Biorad) using amine-coupling chemistry. Briefly, after surface activation the peptide solutions (10 µM in acetate buffer pH 4.0) were injected for 5 min at a flow rate of 30 ml/min, and the remaining activated groups were blocked with ethanolamine, pH 8.0. Bovine serum albumin (BSA) was also immobilized in another parallel flow channel, as a reference protein. The final immobilization levels were similar, about 2500 Resonance Units (1 RU = 1 pg protein/mm²). A fourth surface was prepared using the same immobilization procedure but without addition of the peptide ("empty", reference surface).

The fluidic system of Proteon XPR36 can automatically rotate 90° so that up to six different "analytes" (e.g. different liposomes prepations, or different concentrations of the same preparation) could be injected simultaneously over all the different immobilized molecules.

Preliminary injections were done in order to check for the binding features of the immobilized Aβ species. We thus injected the anti-Aβ antibody 4G8 (Covance) which, as expected bound to both Aβ fibrils and "monomers" (not shown) whereas Congo-Red, a dye specifically recognizing β-sheet-containing species only bound to Aβ fibrils but not "monomers" (not shown).

ProteOn analysis software (BioRad) was used for the fitting of sensorgrams, to obtain the association and dissociation rate constants of the binding (Kon and Koff) and the corresponding K_{D} value. The simplest 1:1 interaction model (Langmuir model) is used at first.

### 2.6.3. Binding characteristics

Before using compound **15** for preparation of curcumin-decorated liposomes **18,** its affinity for Aβ1-42 fibrils was confirmed by SPR studies, using TREG (compound **15** after click reaction with a short PEG chain). The results of this initial confirmatory experiment are presented in Figure 3. Figures 3A and 3C show that the binding of both compounds to Aβ1-42 monomers is characterized by a very fast association and very fast dissociation. A rough estimate of the corresponding Kₒₙ and K_{off} values, obtained after a global fitting of the data, provided K_{D} values of 46 and 23 µM for curcumin and its derivative, respectively. Both compounds showed a higher binding to Aβ1-42 fibrils and, in particular, it appeared a second binding component - additional to the fast association/fast dissociation component also present on monomers. This additional, fibril-specific, component had lower Kₒₙ values (1300 and 163 M⁻¹s⁻¹ for curcumin and its derivative, respectively) and lower K_{off} values (0.03 and 0.001 s⁻¹), resulting in KD values of 25 and 7 µM for curcumin and its derivative, respectively.

No data are available in literature about the actual binding constants of curcumin, as those described here, although it was reported that it inhibits Aβ aggregation in the low µM range [4-7]. Interestingly, the binding properties of the curcumin derivative were even better than those of curcumin itself.

Liposomes **18 (mentioned as 13 in** **Figure 4****)**, as well as plain, i.e. not functionalized, liposomes were flowed over parallel flow channels of the same sensor chip immobilizing Aβ1-42 fibrils, Aβ1-42 "monomers", BSA (to check the specificity of the interaction), and nothing (empty surface). No binding was detected when using plain liposomeson the four sensor surfaces, even at highest curcumin concentrations (light blue lines, Fig. 4). On the contrary, liposomes **18** interacted with immobilized Aβ1-42 species (red lines, Fig.4A-B), in particular with Aβ1-42 fibrils (Fig.4B). A lower binding was observed on BSA (Fig.4C) and an even lower binding was detected on empty surface (Fig.4D).

Figure 5 shows the concentration-dependence of the specific binding of liposomes **18 (mentioned as 13)** to immobilized Aβ1-42 fibrils, i.e. after correction of the signal measured in the reference surface. For these experiments, in particular, we used liposomes with a different density of functionalization (5 or 10%) and injected three concentrations of each of them, to have 100, 300 and 600 nM of exposed curcumin-derivative. The resulting sensorgrams, shown in Figure 5, could not be fitted by a simple 1: 1 interaction model (Langmuir equation) but require more complex interaction models. This is clearly evident by the dissociation phase which cannot be fitted by a mono-exponential curve (as expected for simple interactions), indicating at least two binding components with two different rate constants, one faster and one slower. The fitting of these sensorgrams with a "two-site" model is shown as white lanes in Fig 5, and allowed to estimate the binding constants of the two putative components, highlighting in particular the presence of a component representing about 40% of the binding and characterized by a very low dissociation rate constants (< 3x10⁻⁴ s⁻¹, pseudo-irreversible binding). The corresponding K_{D} values of the exposed curcumin-derivative was calculated to be in the low nM range (2-10 nM). Importantly, the sensorgrams obtained with the different concentrations could not be globally fitted, suggesting that even the binding to this high affinity component is complex and likely involve avidity effects [5].

### 2.7. Confirmation of compound identity - Yield of Click chemistry reaction - liposomes 18

Final confirmation that the click reaction is indeed taking place and liposomes **18** are forming, was provided by identification of the presence of compound **16** in the liposome dispersion after liposomal breakage and HPLC analysis of the resulting mixture. The yield of the click reaction was determined by quantifying the amount of product **16** in weighted quantities of freeze dried liposomal dispersions, and proved to range between 75 and 95 percent.

## Claims

1. Compounds of the general formula (I) and (II)
| | |
|---|---|
| 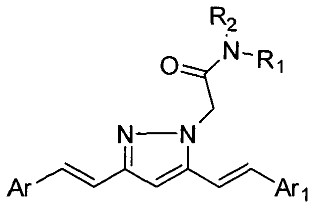 | 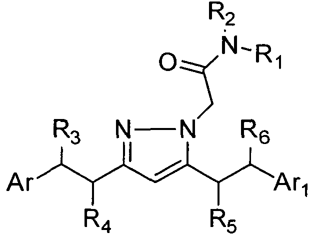 |
| I | II |
wherein R1-R2, equal or different can be hydrogen, alkyl chain, alkenyl chain, alkinyl chain, polymeric chain, cycloalkyl, cycloalkenyl, cycloalkinyl, aryl, heteroaryl or -OH; and Ar-Ar1, equal or different can be 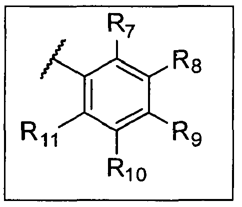 wherein R7, R8, R9, R10 and R11, equal or different can be hydrogen, halogens, alkyl chain, alkenyl chain, alkinyl chain, polymeric chain, cycloalkyl, cycloalkenyl, cycloalkinyl, aryl, heteroaryl, -SR12, -SSR13, -OR14, -NR15R16, where R12, R13, R14, R15, R16 equal or different, represent hydrogen, alkyl chain, alkenyl chain, alkinyl chain, polymeric chain, cycloalkyl, cycloalkenyl, cycloalkinyl, aryl, heteroaryl or -C(X)R17 (except -SSR13) where X could be O, N, S, and R17 represents hydrogen, alkyl chain, alkenyl chain, alkinyl chain, polymeric chain, cycloalkyl, cycloalkenyl, cycloalkinyl, aryl, heteroaryl, -SR18, -OR19, -NR20R21, where R18, R19, R20, R21 equal or different, represent hydrogen alkyl chain, alkenyl chain, alkinyl chain, polymeric chain, cycloalkyl, cycloalkenyl, cycloalkinyl, aryl, heteroaryl.

2. Compounds of general formula (II) wherein R3, R4, R5, R6, equal or different can be hydrogen, halogens, alkyl chain, alkenyl chain, alkinyl chain, polymeric chain, cycloalkyl, cycloalkenyl, cycloalkinyl, aryl, heteroaryl, -SR22, -SSR23, -OR24, - NR25R26, where R22, R23, R24, R25, R26 equal or different, represent hydrogen, alkyl chain, alkenyl chain, alkinyl chain, polymeric chain, cycloalkyl, cycloalkenyl, cycloalkinyl, aryl, heteroaryl or -C(Y)R27 (except -SSR23) where Y could be O, N, S, and R27 represents hydrogen, alkyl chain, alkenyl chain, alkinyl chain, polymeric chain, cycloalkyl, cycloalkenyl, cycloalkinyl, aryl, heteroaryl -SR28, -OR29, - NR30R31, where R28, R29, R30, R31 equal or different, represent hydrogen, alkyl chain, alkenyl chain, alkinyl chain, polymeric chain, cycloalkyl, cycloalkenyl, cycloalkinyl, aryl, heteroaryl.

3. Compounds of the general formula (I) and (II) wherein R1 or R2 equal or different could be
| | |
|---|---|
| 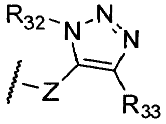 | 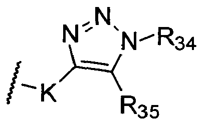 |
Wherein R32, R33, R34, R35 equal or different can be hydrogen, alkyl chain, alkenyl chain, alkinyl chain, polymeric chain, cycloalkyl, cycloalkenyl, cycloalkinyl, aryl, heteroaryl; Z, K can be alkyl chain, alkenyl chain, polymeric chain, cycloalkyl, cycloalkenyl, aryl or heteroaryl.

4. Compounds of formula (I) according to claim 1 wherein R1 = propargyl, R2 = H, Ar=Ar1= 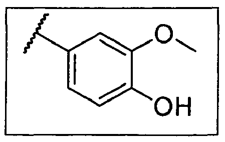 which is: 2-(3,5-bis((E)-4-hydroxy-3-methoxystyryl)-1H-pyrazol-1-yl)-N-(prop-2-yn-1-yl)acetamide

5. Compounds of formula (I) according to claim 1 wherein R1 = H, R2 =
| | | |
|---|---|---|
| 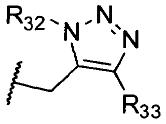 | or | 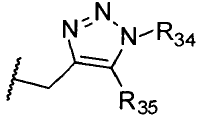 |
Wherein R33/R35 = H and R32/R34 = -CH₂CH₂OCH₂CH₂OCH₂CH₂OH and Ar=Ar1= 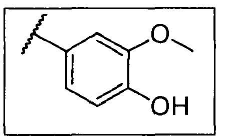 Which is:
2-(3,5-bis((E)-4-hydroxy-3-methoxystyryl)-1H-pyrazol-1-yl)-N-((1-(2-(2-(2-hydroxyethoxy)ethoxy)ethyl)-1H-1,2,3-triazol-4-yl)methyl)acetamide

6. A nanoliposome encapsulating calcein or any other fluorescent or other label or bio-active material or drug, which is functionalised with curcumine derivative on their surface having nano-dimensions and having adequate stability and integrity for in vivo use and which is appropriately designed to maintain the planar structure required for the interaction with Amyloid β (Aβ) and which has very high affinity for Aβ1-42 fibrils, while vesicle size increases as a function of the percent of the functonalization with curcumin and which is stable for up to 20 days.

7. Use of any one of the compounds described in any one of the Claims 1 to 5, as they are or as basis to form derivatives thereof, for the decoration of liposomes according to claim 6.

8. A "click chemistry" and a conventional method of generating a nanoliposome according to Claim 6 , wherein the reaction takes place on liposomes at room temperature (25°C) and the yield of the click reaction ranges between 75% and 95%.

9. A conventional method of generating a nanoliposome according to claim 8 which uses a phospholipid conjugate of the curcumin derivative.

10. A "click chemistry" method of generating a nanoliposome according to Claim 6, wherein the curcumin derivative is produced in situ on the liposomes, which are initially functionalized with an azide-phospholipid, by conjugation of an appropriate precursor of the curcumin derivative opportunely functionalized for the conjugation.
